(19) Europäisches Patentamt
European Patent Office
Office européen des brevets

(11) **EP 3 792 621 B1**

(12) **EUROPÄISCHE PATENTSCHRIFT**

(45) Veröffentlichungstag und Bekanntmachung des Hinweises auf die Patenterteilung:
**31.07.2024  Patentblatt 2024/31**

(21) Anmeldenummer: **19196960.9**

(22) Anmeldetag: **12.09.2019**

(51) Internationale Patentklassifikation (IPC):
*G01N 27/06* (2006.01)    *H01J 35/10* (2006.01)
*F16C 43/02* (2006.01)

(52) Gemeinsame Patentklassifikation (CPC):
**H05G 1/54; F16C 17/02; F16C 33/6692;**
**G01N 27/06; H01J 35/104;** F16C 2233/00;
F16C 2316/10; G01N 33/205; H01J 2235/1086

(54) **MESSVERFAHREN UND MESSVORRICHTUNG FÜR EIN FLÜSSIGMETALL-GLEITLAGER**

MEASURING METHOD AND MEASURING DEVICE FOR A LIQUID-METAL SLIDE BEARING

PROCÉDÉ DE MESURE ET DISPOSITIF DE MESURE POUR UN PALIER LISSE À MÉTAL LIQUIDE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**17.03.2021  Patentblatt 2021/11**

(73) Patentinhaber: **Siemens Healthineers AG**
**91301 Forchheim (DE)**

(72) Erfinder: **Heuermann, Oliver**
**91325 Adelsdorf (DE)**

(74) Vertreter: **Siemens Healthineers**
**Patent Attorneys**
**Postfach 22 16 34**
**80506 München (DE)**

(56) Entgegenhaltungen:
EP-A1- 0 010 539      EP-A1- 3 499 543
EP-B1- 0 010 539      WO-A1-2015/176861
DE-A1- 19 510 068     DE-A1- 19 605 085
JP-A- H05 290 771     US-A1- 2019 090 840

**Beschreibung**

Die Erfindung betrifft ein Messverfahren und Messvorrichtung für ein Flüssigmetall-Gleitlager mit einem rotierenden und einem feststehenden Lagerteil, zwischen welchen sich ein mit Flüssigmetall gefüllter Lagerspalt befindet. Die Messvorrichtung bzw. das Messverfahren dient der Bestimmung des Füllstandes des Flüssigmetall-Gleitlagers mit Flüssigmetall.

Flüssigmetall-Gleitlager (im Folgenden auch einfach als "Gleitlager" oder "Lager" bezeichnet) werden häufig im technischen Bereich der Drehanodenröhren verwendet, insbesondere in hochpreisigen Geräten der Computertomographie und der Angiographie. Sie können aber auch im Bereich der Radiologie, Chirurgie und Mammographie eingesetzt werden. Beispielsweise werden Flüssigmetall-Gleitlager bei Röntgenröhren zur Lagerung der Drehanode verwendet und befinden sich typischerweise im Inneren des Vakuumgehäuses der Röntgenröhre.

Als Flüssigmetall finden in der Regel Gallium-, Indium- oder Zinnlegierungen Verwendung, die bereits bei Raumtemperatur flüssig sind. Das verwendete Flüssigmetall ist bevorzugt eine Legierung aus Gallium, Indium und Zinn. Es wird unter dem Markennamen Galinstan von der Firma Geratherm Medical AG hergestellt und vermarktet.

Heutzutage wird der Flüssigmetallfüllstand eines Gleitlagers mithilfe eines hochenergetischen NDT-Scanners gemessen (NDT: "Non Destructive Testing", dt.: nichtzerstörendes Testen). Typischerweise wird dazu ein Elektronenbeschleunigersystem verwendet, welches Photonen im Energiebereich einiger MeV (Megaelektronenvolt) erzeugt. Ein Beispiel dafür ist ein unter dem Namen "SILAC" bekanntes System (Siemens Industrial Linear Accelerator) mit einer Photonenenergie im Bereich von 6 bis 9 MeV. Diese Prüfung erfolgt zumeist nach dem Füllvorgang, und vor weiteren Fertigungsprozessen des Lagers und ist sowohl zeit- als auch kostenaufwändig. Jedoch ist eine solche Prüfung sehr aussagekräftig und wird im Grunde nur durch die zur Verfügung stehende Bildauflösung eingeschränkt.

Natürlich ist auch einfach möglich, das Gesamtgewicht eines Lagers vor und nach dem Füllvorgang zu erfassen (und es ggf. zur Überprüfung des Füllstandes bzw. zur Reduktion statistischer Fehler noch einmal zu wiegen), bzw. die gemessenen Werte als Referenz für baugleiche Lager zu verwenden. Dies hat jedoch den Nachteil, dass eine solche Messung gravierende Fehler aufweisen kann, da das Gewicht jedes einzelnen Lagerteils und damit auch dessen Abweichung von einem Normgewicht in die Messung eingeht.

Es ist eine Aufgabe der vorliegenden Erfindung, ein alternatives, komfortableres Messverfahren und eine entsprechende Messvorrichtung für Flüssigmetall-Gleitlager (für deren Füllstand) anzugeben, mit dem die oben beschriebenen Nachteile vermieden werden. Insbesondere ist es eine Aufgabe der Erfindung ein Messverfahren bzw. eine Messvorrichtung anzugeben, welche(s) eine Vorbewertung des Füllstandes (z.B. voll, halb voll, leer) erlaubt, bevorzugt nach dem Füllvorgang oder noch während des Füllvorganges im Vakuum.

Diese Aufgabe wird durch ein Messverfahren gemäß Patentanspruch 1, eine Messvorrichtung gemäß Patentanspruch 9 und ein Gerät nach Patentanspruch 12 gelöst.

Das erfindungsgemäße Messverfahren für ein Flüssigmetall-Gleitlager dient zur Messung der Flüssigmetallmenge in dem Flüssigmetall-Gleitlager und umfasst die folgenden Schritte:

- Bereitstellung eines zu vermessenden Flüssigmetall-Gleitlagers, dieses Flüssigmetall-Gleitlager weist dabei zwei Lagerteile auf, zwischen denen das Flüssigmetall angeordnet ist. Typischerweise umfasst ein solches Flüssigmetall-Gleitlager ein inneres Lagerteil und ein äußeres Lagerteil und einem mit Flüssigmetall gefüllten Lagerspalt. Man könnte das Lager auch gedanklich so aufteilen, dass es ein rotierendes und ein feststehendes Lagerteil aufweist, zwischen welchen sich der mit Flüssigmetall gefüllter Lagerspalt befindet. Das umlaufende Lagerteil (also das äußere Lagerteil) kann im Anschluss an den Lagerspalt eine ringförmige Fangnut aufweisen, die austretendes Flüssigmetall aus dem Lagerspalt auffängt. Als Flüssigmetall kann z.B. Galinstan (siehe oben) verwendet werden.

Erfindungsgemäß erfolgt ein Anlegen einer HF-Wechselspannung zwischen elektrischen Kontakten an unterschiedliche Lagerteilen mit einem vorgegebenen Frequenzbereich. Daraufhin wird eine Messung eines HF-Signals an elektrischen Kontakten an unterschiedlichen Lagerteilen durchgeführt. Diese Kontakte können theoretisch dieselben sein, an denen die HF-Wechselspannung angelegt wurde (zumindest wenn die HF-Wechselspannung über einen Widerstand angelegt wurde). Das HF-Signal hängt von der Induktivität des Flüssigmetall-Gleitlagers oder einer mit der Induktivität zusammenhängenden Größe ab.

- Messung der Induktivität des Flüssigmetall-Gleitlagers oder einer mit der Induktivität zusammenhängenden Größe. Dabei ist in der Praxis typischerweise die Induktivität des inneren Lagerteils, des äußeren Lagerteils und des mit Flüssigmetall gefüllten Lagerspalts gemeint. Grundsätzlich genügt es, die Induktivität des Lagerspalts allein zu messen, wenn dies möglich ist. Im Grunde soll hier die Induktivität des Lagerspalts (mit dem Flüssigmetall) allein ermittelt werden (s. Ausführungen zum folgenden Schritt) jedoch ist dies in der Praxis problematisch bis unmöglich.

Oftmals ist es schwierig, die Induktivität des Lagers direkt zu messen. Daher kann man z.B. ausnutzen, dass die Induktivität aus der (komplexen) Impedanz ermittelt werden kann. Diese Impedanz (eine mit der Induktivität zusammenhängenden Größe) kann direkt gemessen werden oder über eine Resonanz (ebenfalls eine mit der Induktivität zusammenhängenden Größe) ermittelt werden.

- Ermittlung der Flüssigmetallmenge in dem Flüssigmetall-Gleitlager basierend auf dem gemessenen HF-Signal.

Ist die Gesamtinduktivität $L_G$ des Flüssigmetall-Gleitlagers bekannt, können die gut bekannten Induktivitäten der Lagerteile $L_{Ti}$, $L_{Ta}$ abgezogen werden. In einem Modell, welches der Realität sehr nahe kommt, entspricht der Aufbau der Induktivitäten der Lagerteile $L_{Ti}$, $L_{Ta}$ und des Lagerspalts $L_S$ einer Reihenschaltung und die Induktivität des Lagerspalts $L_S$ ergibt sich aus $L_S = L_G - (L_{Ti} + L_{Ta})$. Die gemessene Induktivität (also in der Regel die Gesamtinduktivität $L_G$) wird auch als "Streuinduktivität" bezeichnet.

Wie oben bereits angedeutet worden ist, ist die Messung einer Resonanzfrequenz $f_R$ eine praktikable Möglichkeit, um die Gesamtinduktivität $L_G$ des Lagers zu bestimmen. Es gilt dabei

$$f_R = \frac{1}{2\pi\sqrt{LC_K}} \qquad (1)$$

mit der gemessenen Induktivität $L$ (in der Regel die Gesamtinduktivität $L_G$) und der bekannten Resonanzkreiskapazität $C_K$. Die Resonanzkapazität als Bauteil wird eigens berechnet und gebaut (liegt im Bereich 10pF) und mit einer Präzisions-Messbrücke überprüft. Oft sind solche Referenzkapazitäten auch Bestandteil der Messbrückenkalibrierung. Diese Ausführung des Verfahrens wird weiter unten genauer erläutert. Es ist aber auch möglich, Impedanzminima oder -maxima des Lagers mit einem Impedanzanalysator zu bestimmen.

Hierzu ist zu beachten, dass die Streuinduktivität eines Flüssigmetall-Gleitlagers sehr klein ist (ca. einige Zehn bis Hundert nH). Daher liegt die Resonanzfrequenz typischerweise im VHF bis UHF-Bereich (z.B. zwischen 150 MHz und 2000 MHz).

Zur Ermittlung der Flüssigmetallmenge wird das Lager als elektrischer Leiter mit einer vom Füllgrad abhängigen Gesamtinduktivität angenommen. Dabei wird von dem Gedankenmodell ausgegangen, dass das Flüssigmetall im Lagerspalt mit einer Parallelschaltung mehrerer Strukturelemente vergleichbar ist. Als Strukturelement kann ein elementarer Strompfad betrachtet werden, der vom Außenlager (äußeres Lagerteil) über den Lagerspalt zum Innenlager (inneres Lagerteil) führt. Ein Strukturelement kann als eigenständige Induktivität angesehen werden, wobei ein Strukturelement auch zusätzlich andere Eigenschaften haben kann (wie z.B. zusätzlich einen ohmschen Anteil). Je höher der Flüssigmetallstand im Lagerspalt, desto mehr Strukturelemente sind parallelgeschaltet. Je mehr Strukturelemente parallelgeschaltet sind, desto geringer ist die resultierende Induktivität des Lagerspalts $L_S$ und damit auch die Streuinduktivität (gemessene Induktivität bzw. Gesamtinduktivität). Die Induktivität des Lagerspalts $L_S$ kann also mit der Formel

$$L_S = \frac{1}{\frac{1}{L_1} + \frac{1}{L_2} + ... + \frac{1}{L_n}} \qquad (2)$$

als eine aus den Induktivitäten der Strukturelemente $L_i$ zusammengesetzte Induktivität betrachtet werden. Diese $L_i$ können alle gleich sein, in der Realität sind diese Li aufgrund der Bauform des Lagers jedoch zumeist unterschiedlich.

Wird das Lager bei einer Messung immer gleich ausgerichtet (und befinden sich keine Luftblasen im Flüssigmetall) kann angenommen werden, dass die $L_i$ von $L_1$ an bis zu einem bestimmten Wert n parallel geschaltet sind. Der Wert "n" ist dabei ein Maß für den Füllstand. Ist der Füllstand niedrig, also die Anzahl der parallel geschalteten Strukturelemente, so ist auch n niedrig und umgekehrt. Der Füllstand kann also nach der Formel

$$\frac{1}{L_S} = \sum_{1}^{n} \frac{1}{L_i} \qquad (3)$$

ermittelt werden, wobei diese Formel nach n aufgelöst werden muss. Bei gleichartigen $L_i$ ist dies einfach möglich, bei jeweils unterschiedlichen $L_i$ kann z.B. eine Tabelle der (bekannten) Werte für $L_i$ verwendet werden.

Es wäre auch möglich, an Stelle der $L_i$ eine kontinuierliche Funktion L(h) zu verwenden, wobei h die Höhe des Füllstandes oder eine Höhenkoordinate relativ zum Füllstand sein kann. Eine erfindungsgemäße Messvorrichtung dient zur Vermessung eines Flüssigmetall-Gleitlagers, also der Vermessung der Flüssigmetallmenge im Lager. Das Flüssigmetall-Gleitlager umfasst zwei Lagerteile, zwischen denen das Flüssigmetall angeordnet ist, insbesondere einem inneren Lagerteil, einem äußeren Lagerteil und einem mit Flüssigmetall gefüllten Lagerspalt. Die Messvorrichtung umfasst die folgenden Komponenten.

- Eine Messeinheit ausgelegt zur Messung der Induktivität des Flüssigmetall-Gleitlagers oder einer mit der Induktivität zusammenhängenden Größe.

- Eine Ermittlungseinheit ausgelegt zur Ermittlung der Flüssigmetallmenge in dem Flüssigmetall-Gleitlager basierend auf der Induktivität oder der mit der Induktivität zusammenhängenden Größe.

Ein Flüssigmetall-Gleitlager umfasst zwei Lagerteile, zwischen denen das Flüssigmetall angeordnet ist, bevorzugt ein inneres Lagerteil und ein äußeres Lagerteil, zwischen welchen sich ein mit Flüssigmetall gefüllter Lagerspalt befindet. Das Flüssigmetall-Gleitlager ist dabei zu einem Anschluss einer Messeinheit einer erfindungsgemäßen Messvorrichtung ausgestaltet. Eines der Lagerteile umfasst bevorzugt einen elektrischen Kontakt zum Anschluss einer HF-Einheit/HF-Generator der Messvorrichtung und das andere Lagerteil einen elektrischen Kontakt zum Anschluss einer Messeinheit der Messvorrichtung.

Eine Röntgenröhre umfasst eine Drehanode, welche mittels eines Flüssigmetall-Gleitlagers drehbar gelagert ist.

Ein erfindungsgemäßes Gerät, insbesondere ausgelegt zur Untersuchung mittels Röntgenstrahlung, umfasst eine erfindungsgemäße Messvorrichtung und ein Flüssigmetall-Gleitlager. Das Gerät ist bevorzugt ein medizintechnisches Gerät, z.B. ein Röntgengerät oder ein Computertomographiegerät.

Zusammenfassend lässt sich sagen, dass eine Besonderheit der Erfindung ist, dass das Flüssigmetall-Gleitlager aus elektrotechnischer Sicht betrachtet wird und zwar als eine mit Flüssigmetall gefüllte Koaxialanordnung. Die Streuparameterwerte des Lagers werden erfindungsgemäß elektrisch erfasst, um daraus auf den Flüssigmetallfüllstand zu schließen. Eine Kalibrierung der Messmethode kann z.B. erfolgen, indem ein, mit einem Elektronenbeschleunigersystem-Scan, insbesondere mit einem SILAC-Scan (Siemens Industrial Linear Accelerator) abgesichertes, vollständig gefülltes Lager vermessen wird. Dieser Wert des Füllstandes stellt dann die Referenz für die folgenden vergleichenden Messungen dar.

Ein Großteil der zuvor genannten Komponenten der Messvorrichtung, können ganz oder teilweise in Form von Softwaremodulen in einem Prozessor einer entsprechenden Messvorrichtung realisiert werden. Eine weitgehend softwaremäßige Realisierung hat den Vorteil, dass auch schon bisher verwendete Messvorrichtungen auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in ein Rechensystem einer Messvorrichtung ladbar ist, mit Programmabschnitten, um alle Schritte des erfindungsgemäßen Verfahrens auszuführen, wenn das Programm in dem Rechensystem ausgeführt wird. Ein solches Computerprogrammprodukt kann neben dem Computerprogramm gegebenenfalls zusätzliche Bestandteile wie z.B. eine Dokumentation und/oder zusätzliche Komponenten auch Hardware-Komponenten, wie z.B. Hardware-Schlüssel (Dongles etc.) zur Nutzung der Software, umfassen.

Zum Transport zum Rechensystem und/oder zur Speicherung an oder in dem Rechensystem kann ein computerlesbares Medium, z.B. ein Memorystick, eine Festplatte oder ein sonstiger transportabler oder fest eingebauter Datenträger dienen, auf welchem die von einem Rechensystem einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Die Erfindung umfasst somit auch ein Computerprogrammprodukt mit einem Computerprogramm, welches direkt in eine Speichereinrichtung einer Messvorrichtung oder eines Gerätes ladbar ist, mit Programmabschnitten, um wesentliche Schritte des erfindungsgemäßen Verfahrens (die Ermittlung) auszuführen, wenn das Computerprogramm in der Messvorrichtung oder dem Gerät ausgeführt wird.

Die Erfindung umfasst somit auch ein computerlesbares Medium, auf welchem von einer Rechnereinheit einlesbare und ausführbare Programmabschnitte gespeichert sind, um alle Schritte des erfindungsgemäßen Verfahrens auszuführen, wenn die Programmabschnitte von der Rechnereinheit ausgeführt werden.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

In der Theorie ist es im Hinblick auf das Verfahren im Grunde egal, wo die Messung erfolgt (z.B. wo ein HF-Wechselstrom fließt und wo der Messabgriff liegt), sofern nur genügend Stromwege durch den Lagerspalt führen. In der Praxis sind dabei jedoch oft Grenzen gesetzt. Eine dieser Grenzen ist, dass das Flüssigmetall sich im Inneren des Lagers befindet und im Grunde gar nicht direkt kontaktierbar ist, ansonsten wäre es ideal, einen Messabgriff direkt an oder über dem Lagerspalt anzuordnen, also direkt am Flüssigmetall. Dies ist aber in der Regel praktisch unmöglich.

Deshalb werden in der Praxis in der Regel die Induktivitäten von Außen- und Innenlager mit in die Messung einbezogen. Die Werte für die Induktivitäten dieser Lagerteile $L_{Ti}$, $L_{Ta}$ sind aufgrund deren engen geometrischen Toleranzen sehr stabil. Die Breite des Lagerspaltes zwischen Außen- und Innenlager liegt typischerweise im μm-Bereich. Dieser enge Spalt sollte bei einem optimal gefüllten Lager zur Gänze mit Flüssigmetall gefüllt sein, damit das Lager unter Betriebsbedingungen ausreichend tragfähig ist.

Eine bevorzugte Messvorrichtung umfasst dazu eine HF-Einheit ausgelegt zum Anlegen einer HF-Wechselspannung zwischen elektrischen Kontakten an unterschiedliche Lagerteilen mit einem vorgegebenen Frequenzbereich, eine Messeinheit ausgelegt zur Messung eines HF-Signals an einem elektrischen Kontakt (oder an zwei elektrischen Kontakten an unterschiedlichen Lagerteilen) und eine Ermittlungseinheit ausgelegt zur Ermittlung der Flüssigmetallmenge basierend auf dem gemessenen HF-Signal. Die HF-Einheit ist hier nicht zwingend notwendig und kann durchaus weggelassen werden. In diesem Falle müsste jedoch für eine Messung eine externe HF-Einheit verwendet werden.

Es wird darauf hingewiesen, dass das Anlegen einer Wechselspannung aufgrund des ohmschen Gesetzes gleichbedeutend mit der Einbringung eines Wechselstroms in das Lager ist. Zur Messung der HF-Wechselspannung an denselben elektrischen Kontakten kann ein ohmscher Widerstand in Reihe mit der Quelle der HF-Spannung geschaltet werden. Der Widerstand trennt die Signalquelle vom zu testenden Lager. In der Serienresonanz erzeugt der Stromfluss durch den Realanteil einen Spannungsabfall über dem Widerstand. Das Spannungsminimum gegenüber der Signalquelle kann dann gemessen werden. Bevorzugt wird dann über diesem Widerstand oder über der Reihenschaltung Widerstand & HF-Einheit die Spannung gemessen. Die Kontakte können aber theoretisch auch so platziert werden, dass der ohmsche Widerstand des Lagerspalts ausgenutzt wird. Der eingebrachte HF-Wechselstrom muss dabei den mit Flüssigmetall gefüllten Lagerspalt durchdringen. Dazu sollte ein Potentialunterschied, zwischen den bei Lagerteilen bestehen. Der HF-Wechselstrom sollte auch möglichst viel Flüssigmetall durchdringen, um einen Spannungsabfall zu erzeugen. Dies ist vorteilhaft, da dieser den induktiven Blindwiderstand widerspiegelt, aus dem man die Induktivität leicht bestimmen kann.

Gemäß einem bevorzugten Messverfahren erfolgt eine Ermittlung einer Resonanzfrequenz aus der Messung und anschließend eine Ermittlung der Flüssigmetallmenge basierend auf der Resonanzfrequenz. Dies kann z.B. nach der Formel (1) erfolgen, wobei die Formel nach der gemessenen Induktivität L (in der Regel ist dies die Gesamtinduktivität $L_G$) aufgelöst wird und die (konstanten) Induktivitäten der Lagerteile abgezogen werden. Es gilt danach, dass $L_S$ antiproportional zum Quadrat der Resonanzfrequenz $f_R$ ist. Letztendlich kann die gemessene Resonanzfrequenz $f_R$ zur Ermittlung der Flüssigmetallmenge und damit des Flüssigmetallstandes im Lagerspalt auch mit einem Wert einer Wertetabelle oder einer Kurve verglichen werden.

Gemäß einem bevorzugten Messverfahren erfolgt eine Ermittlung eines Minimums (bei Serienresonanz) oder eines Maximums (bei Parallelresonanz) der Lagerimpedanz aus der Messung und anschließend eine Ermittlung der Flüssigmetallmenge basierend auf der Lagerimpedanz.

Gemäß einem bevorzugten Messverfahren erfolgt eine Ermittlung der Flüssigmetallmenge basierend auf einem Verhältnis der Amplitude und der Phase von dem gemessenen HF-Signal, und bevorzugt der angelegten HF-Wechselspannung, über ein Frequenzintervall (z.B. von 0 bis 2000MHz). Beispielsweise könnte die Kurve eines Bodeplotters analysiert werden oder generell Bodeplotterergebnisse, also das Verhältnis der Amplitude und der Phase von einem Ein- und einem Ausgangssignal über besagtes Frequenzintervall (z.B. 150MHz bis 2 GHz). Die obere Frequenz des Frequenzintervalls kann aber auch größer sein als 1GHz.

Gemäß einem bevorzugten Messverfahren ist die Frequenz der HF-Wechselspannung größer als 50 MHz, bevorzugt größer als 100 MHz, jedoch insbesondere kleiner als 2 GHz. Hierzu sollte beachtet werden, dass nicht zwingend eine einzige Frequenz (also eine Wechselspannung mit einer einzigen Frequenz) angelegt werden muss. In der Praxis ist es nicht möglich eine einzige Frequenz einzustrahlen, sondern stets ein Frequenzintervall einer bestimmten Breite. In der Praxis wird die zentrale Frequenz eines solchen Intervalls als Hauptfrequenz angesehen. Diese Hauptfrequenz ist hier gemeint. Es kann durchaus eine HF-Wechselspannung mit mehreren Hauptfrequenzen (Moden) eingestrahlt werden, um unterschiedliche Resonanzen im Lager anzuregen. Dazu ist insbesondere bevorzugt, dass mindestens zwei Hauptfrequenzen oder sogar mindestens 5 Hauptfrequenzen eingestrahlt werden (bzw. die HF-Wechselspannung diese Hauptfrequenzen aufweist).

Gemäß einem bevorzugten Messverfahren wird aus der gemessenen Induktivität des Flüssigmetall-Gleitlagers oder einer mit der Induktivität zusammenhängenden Größe zunächst die Induktivität $L_{EM}$ des Flüssigmetallanteils ermittelt. Dabei können z.B. die vorbekannten Induktivitäten des inneren Lagerteils und des äußeren Lagerteils von der gemessenen Induktivität abgezogen werden. Danach wird mit vorbestimmten Werten $L_i$ auf Basis der Formel (3) (

$$\frac{1}{L_S} = \sum_1^n \frac{1}{L_i}$$

) die Größe n ermittelt. Es ist aber auch ein integraler Ansatz möglich, bei dem keine Summe, sondern eine Funktion zur Berechnung von $L_S$ verwendet wird.

Hier bedeutet also ein niedriger Füllgrad, dass weniger parallel geschaltete Strukturelemente existieren und ein hoher Füllgrad, dass mehr parallel geschaltete Strukturelemente existieren, wobei die Strukturelemente rein theoretische Elemente zur Beschreibung des Lagers darstellen.

Gemäß einem bevorzugten Messverfahren erfolgt vor der Ermittlung der Flüssigmetallmenge eine Kalibrierung an einem zum vermessenen Flüssigmetall-Gleitlager baugleichen Flüssigmetall-Gleitlager. Dieses baugleiche Lager ("Referenzlager") ist insbesondere vollständig gefüllt. Für die Kalibrierung wird das Referenzlager (das baugleiche Flüssigmetall-Gleitlager) bevorzugt mittels einer Durchleuchtung vermessen, besonders bevorzugt mittels eines Elektronenbeschleunigersystem-Scans. Das Referenzlager kann dann mit dem erfindungsgemäßen Messverfahren vermessen werden und der so ermittelte Wert der Streuinduktivität stellt dann die Referenz für ein Maximum der Füllmenge für alle vergleichenden Messungen dar. Es können selbstverständlich auch verschiedene Referenzlager mit unterschiedlichen Füllständen vermessen werden, nämlich zunächst genau (z.B. SILAC) zur Ermittlung des genauen Füllstandes, und dann mit dem erfindungsgemäßen Verfahren zum Erhalt einer Referenzgröße. Wird nun ein unbekanntes Lager mit dem erfindungsgemäßen Verfahren vermessen, können diese gemessenen Werte mit den Referenzwerten verglichen werden, um ein Maß für die Füllhöhe des vermessenen Lagers zu erhalten.

Gemäß einem bevorzugten Messverfahren erfolgt basierend auf der Ermittlung der Flüssigmetallmenge an dem Flüssigmetall-Gleitlager eine weitere Messung der Flüssigmetallmenge, insbesondere wenn die ermittelte Flüssigmetallmenge unter einem vorher festgelegten Grenzwert liegt. Diese weitere Messung erfolgt dabei mittels eines anderen Messverfahrens, bevorzugt mittels einer Durchleuchtung des Flüssigmetall-Gleitlagers, besonders bevorzugt mittels eines Elektronenbeschleunigersystem-Scans. Auf diese Weise kann zunächst mit dem erfindungsgemäßen Messverfahren sehr schnell und einfach festgestellt werden, ob ein Lager optimal gefüllt ist und bei Feststellen einer nicht optimalen Füllung das Lager mit einer genaueren, aber aufwändigeren Messmethode überprüft werden. Mithilfe des erfindungsgemäßen Messverfahrens kann also z.B. eine Vorauswahl getroffen werden, ob sich ein Elektronenbeschleunigersystem-Scan lohnt oder nicht. Wenn das erfindungsgemäßen Messverfahren im Vakuum des Gleitlagerfüllstandes angewendet würde, könnte eine Aussage getroffen werden, ob der Füllvorgang erfolgreich war oder ob er fortgeführt werden muss.

Eine bevorzugte Messvorrichtung umfasst eine Steuereinheit zur Steuerung eines Messgeräts, welches ein anderes Messverfahren als die Messvorrichtung durchführt, bevorzugt mittels einer Durchleuchtung des Flüssigmetall-Gleitlagers, besonders bevorzugt mittels eines Elektronenbeschleunigersystem-Scans. Durch die Messvorrichtung wird z.B. eine Messung durch das Messgerät gestartet und/oder ausgewertet.

Gemäß einer bevorzugten Ausführungsform werden im Rahmen der Ermittlung der Resonanzfrequenz neben der reinen Ermittlung der Resonanzfrequenz noch weitere Informationen aus den Messergebnissen abgeleitet. Bevorzugt wird dabei die Kurvenform der Resonanzkurve weiter untersucht, z.B. nach weiteren Resonanzfrequenzen (Nebenmaxima) oder Abweichungen der Kurvenform von einer reinen Resonanzkurve mit einer einzigen Resonanzfrequenz. Zudem kann die Amplitude der Resonanzfrequenz untersucht werden. Damit kann, z.B. nach Vergleich mit Untersuchungsergebnissen an Lagern, die mit unterschiedlichen Flüssigmetallmengen gefüllt waren, festgestellt werden, an welchen Stellen Flüssigmetall fehlt. Dies liegt daran, dass bei einem realen Lager der Querschnitt in den verschiedenen Lagerbereichen unterschiedlich ist. Es bilden sich damit unterschiedliche resultierende Induktivitäten $L_i$ (s. Formel 2) für die unterschiedlichen Bereiche aus. Dies liegt im Wesentlichen daran, dass der Strom an unterschiedlichen Stellen des Lagers unterschiedlich große Flächen überwinden muss. Es kann also z.B. eine Trennung von Fehlstellen im Axiallager gegenüber Fehlstellen im Radiallager erfolgen. Bevorzugt erfolgt im Rahmen der Ermittlung eine Fouriertransformation der Resonanzkurve.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:

Figur 1 einen Längsschnitt durch eine Drehanodenröntgenröhre mit einem Flüssigmetall-Gleitlager für die Drehanode in teilweise geschnittener Darstellung,

Figur 2 einen Längsschnitt durch ein Flüssigmetall-Gleitlager,

Figur 3 ein Schaltbeispiel für ein Modell der Erfindung,

Figur 4 ein Blockdiagramm für einen möglichen Ablauf eines erfindungsgemäßen Verfahrens und

Figur 5 eine Darstellung von möglichen HF-Signalen.

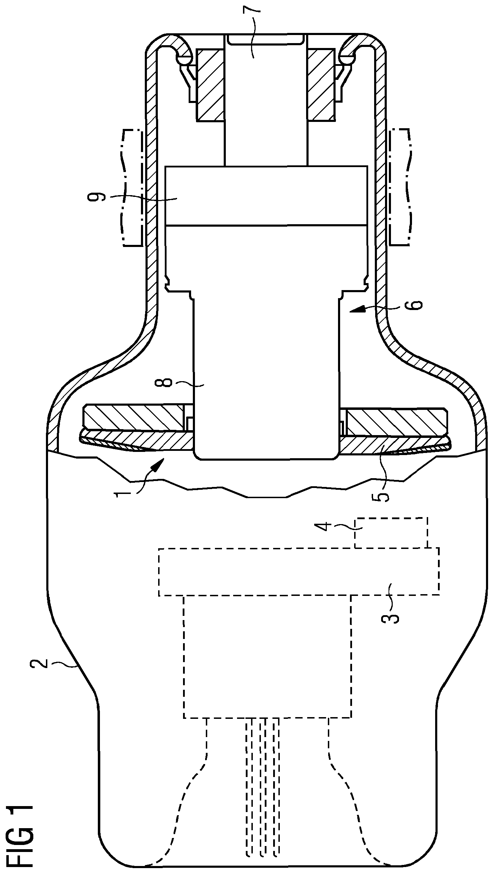

In der Figur 1 ist eine Drehanoden-Röntgenröhre dargestellt, die eine Drehanode 1 aufweist, die in einem Vakuumkolben 2 untergebracht ist. Der Vakuumkolben 2 enthält außerdem noch in an sich bekannter Weise eine Kathode 3, die in einem Fokuskopf 4 einen oder mehrere hier nicht sichtbare Elektronenemitter enthält.

Um die drehbare Lagerung der Drehanode 1 zu gewährleisten, ist ein Flüssigmetall-Gleitlager 6 vorgesehen, das als inneres Lagerteil 7 eine fest mit dem Vakuumkolben 2 verbundene Lagerungsachse aufweist. An dem rotierenden äußeren Lagerteil 8 ist der Anodenteller 5 der Drehanode 1 fest angebracht. Ein Lagerflansch 9 verhindert ein Austreten von Flüssigmetall aus dem Lager.

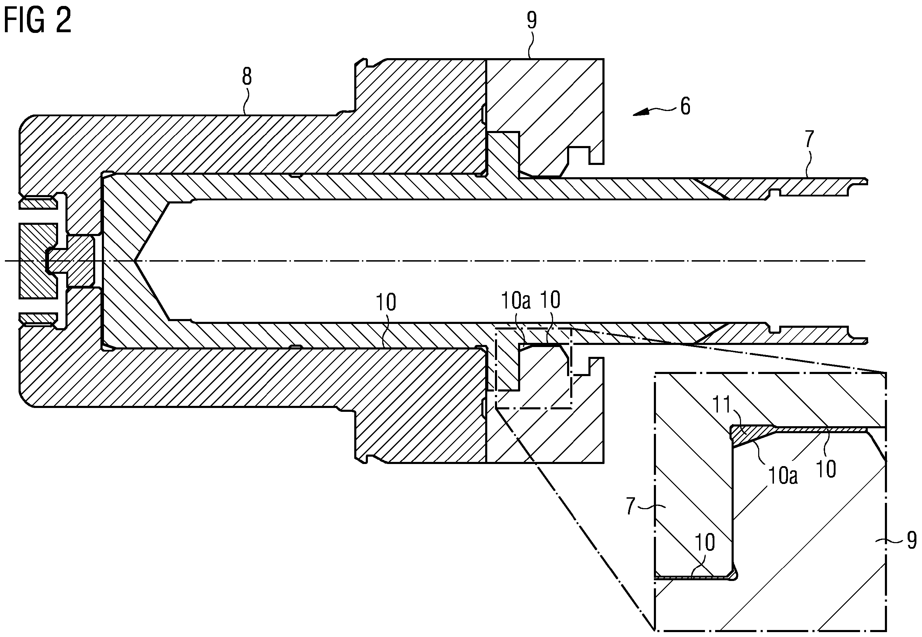

Figur 2 zeigt einen Längsschnitt durch ein Flüssigmetall-Gleitlager 6, wie es zum Beispiel in einer Drehanode nach Figur 1 eingebaut sein könnte. Das Flüssigmetall-Gleitlager 6 umfasst ein inneres Lagerteil 7, ein äußeres Lagerteil 8 und einen Lagerspalt 10 zwischen diesen Lagerteilen 7, 8. Ein Lagerflansch 9 verhindert ein Austreten von Flüssigmetall 11 aus dem Lagerspalt 10. Rechts unten ist eine Vergrößerung des Lagerspalts 10 zu sehen, der mit Flüssigmetall 11 gefüllt ist, d.h. einem Metall, das bereits bei Raumtemperatur flüssig ist. Der Lagerspalt 10 ist an seinen Engstellen z.B. einige $\mu$m breit weist jedoch hier auch dickere Bereiche auf, die als Reservoir 10a für Flüssigmetall 11 dienen.

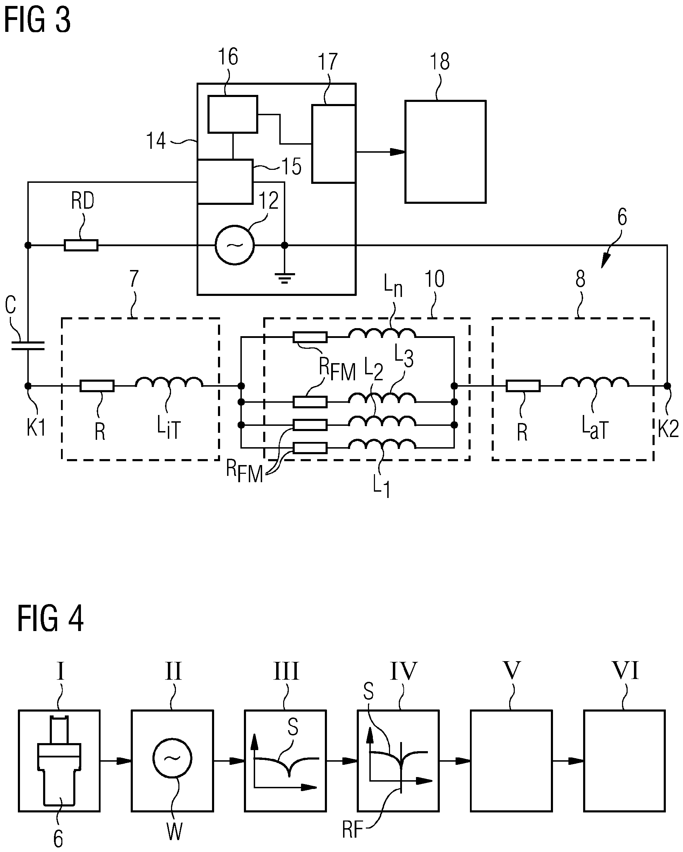

Figur 3 zeigt ein Schaltbeispiel für ein Modell der Erfindung. Die eingezeichneten elektronischen Bauteile wie Widerstände R, Kapazitäten C und Induktivitäten $L_{iT}$, $L_{aT}$, $L_1$, $L_2$, $L_3$, $L_n$ liegen in der Regel nicht als reale Bauteile vor, sondern stellen elektrische Eigenschaften ("Strukturelemente") des Flüssigmetall-Gleitlagers 6 dar, wie es z.B. in Figur 2 gezeigt ist.

Unten ist das Flüssigmetall-Gleitlagers 6 in Form eines schematischen Ersatzschaltbildes dargestellt. Das Flüssigmetall-Gleitlager 6 weist eine Reihe von Reihenschaltungen aus einer Induktivität $L_{iT}$, $L_{aT}$, $L_1$, $L_2$, $L_3$, $L_n$ und einem ohmschen Widerstand R der Lagerteile 7, 8 und $R_{FM}$ des Flüssigmetalls 11 (der Widerstand spielt hier nur eine untergeordnete Rolle).

Diese Reihenschaltungen können auch als "Strukturelemente" bezeichnet werden, da sie nicht wirklich vorhanden sind, sondern elektrische Eigenschaften des Lagers beschreiben. Das innere Lagerteil 7 hat in dem Ersatzschaltbild eine Induktivität $L_{iT}$ und das äußere Lagerteil 8 hat in dem Ersatzschaltbild eine Induktivität $L_{aT}$. Der Lagerspalt 10 hat als Ersatzschaltbild eine Parallelschaltung von gleichen oder unterschiedlichen Induktivitäten $L_1$, $L_2$, $L_3$, $L_n$. Je höher der Flüssigmetallanteil im Lagerspalt 10, desto mehr Induktivitäten $L_1$, $L_2$, $L_3$, $L_n$ liegen dort vor. Das innere Lagerteil 7, das äußere Lagerteil und der Lagerspalt 10 bilden eine Reihenschaltung bezüglich der Ersatzschaltbilder.

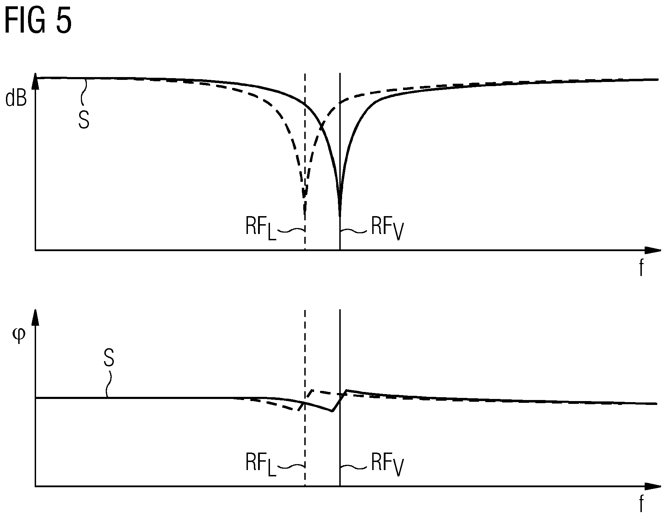

Oben ist eine Messvorrichtung 14 gemäß einer bevorzugten Ausführungsform dargestellt. Die Messvorrichtung 14 umfasst eine HF-Einheit 12 (z.B. einen HF-Generator 12) ausgelegt zum Anlegen einer HF-Wechselspannung zwischen elektrischen Kontakten K1, K2 an unterschiedlichen Lagerteilen 7, 8 (an dem Kontakt K1 am äußeren Lagerteil 8 und dem Kontakt K2 am inneren Lagerteil 7) mit einem vorgegebenen Einstrahlungsfrequenzbereich, eine Messeinheit 15 ausgelegt zur Messung eines HF-Signals S (s. z.B. Figur 5) an zwei elektrischen Kontakten K1, K2 an den unterschiedlichen Lagerteilen 7, 8 und eine Ermittlungseinheit 16 ausgelegt zur Ermittlung der Flüssigmetallmenge basierend auf dem gemessenen HF-Signal S.

Zusätzlich umfasst die Messvorrichtung noch eine Steuereinheit 17. Diese Steuereinheit 17 steuert ein Messgerät 18, z.B. ein Elektronenbeschleunigersystem, insbesondere einen SILAC-Scanner, welches ein anderes Messverfahren als die Messvorrichtung 14 durchführt. Auf diese Weise kann automatisch z.B. eine Durchleuchtung eines vermessenen Flüssigmetall-Gleitlagers 6 erfolgen, wenn die Vermessung Probleme des Flüssigmetallanteils anzeigt.

Der Dämpfungswiderstand RD (z.B. mit 100 Ohm) trennt die Signalquelle (HF-Einheit 12) vom Flüssigmetall-Gleitlager 6 und der Messvorrichtung 14. In der Serienresonanz erzeugt der Stromfluss durch den Realanteil einen Spannungsabfall über diesem Dämpfungswiderstand RD. Das Spannungsminimum gegenüber der Signalquelle kann gemessen werden. Bei Parallelresonanz steigt der Widerstand, es entsteht ein Stromminimum, welches wiederum als Spannungsabfall über dem Dämpfungswiderstand RD in Erscheinung tritt. Bei praktischen HF-Anwendungen arbeitet man oft auch mit einem Dämpfungswiderstand RD von 50 Ohm.

Es wird darauf hingewiesen, dass dieses Schaltbild, insbesondere die Teile, die ein Ersatzschaltbild darstellen, nur skizzenhaft dargestellt ist. So könnten z.B. noch Leitungsimpedanzen, Impedanzen von Tastköpfen oder Abschlusswiderstände hinzugefügt werden. Zur besseren Übersicht wurden all diese Komponenten hier weggelassen.

Da die Höhe des Flüssigmetallstandes im Lagerspalt 10 einer Anzahl von N parallelgeschalteten Induktivitäten $L_1$, $L_2$, $L_3$, $L_n$ entspricht und die Resonanzfrequenz von diesen Induktivitäten $L_1$, $L_2$, $L_3$, $L_n$ abhängig ist, kann aus der Lage der Resonanzfrequenz ermittelt werden. Je höher der Füllstand, desto größer n, desto größer die Resonanzfrequenz. Um die Messung zu bestätigen oder den genauen Grad der Füllung zu ermitteln kann die Steuereinheit 17 nun das Messgerät 18 anschalten und eine (langsame, aber dafür genauere) Messung mit diesem Messgerät 18 durchführen.

In der Praxis ist die Gesamtinduktivität $L_{iT}$, $L_{aT}$, $L_1$, $L_2$, $L_3$, $L_n$ eines Flüssigmetall-Gleitlagers 6 sehr klein (nH-Bereich). Daher muss die HF-Einheit 12 mit VHF-Frequenzen oder UHF-Frequenzen betrieben werden.

Figur 4 zeigt ein Blockdiagramm für einen möglichen Ablauf eines erfindungsgemäßen Messverfahrens für ein Flüssigmetall-Gleitlager 6.

In Schritt I erfolgt eine Bereitstellung eines zu vermessenden Flüssigmetall-Gleitlagers 6 (s. z.B. Figur 2), welches zwei Lagerteile 7, 8 aufweist, zwischen denen das Flüssigmetall 11 angeordnet ist. Eine entsprechende Messvorrichtung wird z.B. an das Flüssigmetall-Gleitlager 6 angeschlossen wie in Figur 3 gezeigt.

In Schritt II wird eine HF-Wechselspannung zwischen elektrischen Kontakten K1, K2 an unterschiedliche Lagerteile 7, 8 angelegt. Die HF-Wechselspannung hat dabei einen vorgegebenen Einstrahlungsfrequenzbereich.

In Schritt III erfolgt eine Messung eines HF-Signals S an elektrischen Kontakten K1, K2 unterschiedlicher Lagerteile 7, 8.

In Schritt IV erfolgt eine Ermittlung einer Resonanzfrequenz RF aus dem gemessenen HF-Signal S.

In Schritt V erfolgt eine Ermittlung der Flüssigmetallmenge basierend auf der Resonanzfrequenz RF, z.B. unter Berücksichtigung der

$$\text{Formeln (1)} \quad f_R = \frac{1}{2\pi\sqrt{L_G C_K}}$$

und

$$(3) \quad \frac{1}{L_S} = \sum_1^n \frac{1}{L_i} \, .$$

In Schritt VI erfolgt basierend auf der Ermittlung der Flüssigmetallmenge (hier liegt z.B. die ermittelte Flüssigmetallmenge unter einem vorher festgelegten Grenzwert) an dem Flüssigmetall-Gleitlager 6 eine weitere Messung der Flüssigmetallmenge. Diese Messung erfolgt dabei mittels eines anderen Messverfahrens, z.B. mittels einer Durchleuchtung des Flüssigmetall-Gleitlagers 6, die mittels eines SILAC-Scans durchgeführt werden kann.

Figur 5 zeigt eine Darstellung von möglichen HF-Signalen aufgetragen gegen die Frequenz f. Oben ist die Amplitude des Signals im Hinblick auf seine Dämpfung in dB angegeben, unten ein Bodeplot, welcher die Phase zum Eingangssignal (HF-Wechselspannung) darstellt.

Gezeigt sind hier jeweils zwei HF-Signale S. Eines bei vollem Flüssigmetall-Gleitlager 6 (durchgezogene Linie) und eines bei leerem (gestrichelt). Deutlich erkennbar ist die Resonanzfrequenz $RF_V$ bei vollem Flüssigmetall-Gleitlager 6 höher als die Resonanzfrequenz $RF_L$ bei leerem Flüssigmetall-Gleitlager 6. Der Grad der Abweichung von der Resonanzfrequenz $RF_V$ bei vollem Flüssigmetall-Gleitlager 6 ist ein Maß für die Flüssigmetallmenge im Flüssigmetall-Gleitlager 6. Eine genaue Korrelation kann durch Referenzmessungen an Flüssigmetall-Gleitlagern 6 mit bekanntem Füllstand erreicht werden.

Es wird abschließend noch einmal darauf hingewiesen, dass es sich bei den vorhergehend detailliert beschriebenen Messverfahren bzw. der Messvorrichtung sowie bei dem dargestellten Flüssigmetall-Gleitlager lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einheit" und "Vorrichtung" nicht aus, dass die betreffenden Komponenten aus mehreren zusammenwirkenden Teil-Komponenten bestehen, die gegebenenfalls auch räumlich verteilt sein können.

**Patentansprüche**

1. Messverfahren für ein Flüssigmetall-Gleitlager (6), umfassend die Schritte:

   - Bereitstellung eines zu vermessenden Flüssigmetall-Gleitlagers (6), welches zwei Lagerteile (7, 8) aufweist, zwischen denen das Flüssigmetall (11) angeordnet ist,
   - Anlegen einer HF-Wechselspannung zwischen elektrischen Kontakten (K) an unterschiedliche Lagerteilen (7, 8) mit einem vorgegebenen Frequenzbereich,
   - Messung eines HF-Signals (S) an elektrischen Kontakten (K1, K2) an unterschiedlichen Lagerteilen (7, 8), wobei das HF-Signal (S) von der Induktivität des Flüssigmetall-Gleitlagers (6) oder einer mit der Induktivität zusammenhängenden Größe abhängt,
   - Ermittlung der Flüssigmetallmenge basierend auf dem gemessenen HF-Signal (S).

2. Messverfahren nach Anspruch 1, umfassend die Schritte:

   - Ermittlung einer Resonanzfrequenz (RF, $RF_L$, $RF_V$) aus dem HF-Signal (S),
   - Ermittlung der Flüssigmetallmenge basierend auf der Resonanzfrequenz (RF, $RF_L$, $RF_V$).

3. Messverfahren nach einem der vorangehenden Ansprüche, umfassend die Schritte:

- Ermittlung eines Minimums oder eines Maximums der Lagerimpedanz aus der Messung,
- Ermittlung der Flüssigmetallmenge basierend auf der Lagerimpedanz.

4. Messverfahren nach einem der vorangehenden Ansprüche, wobei eine Ermittlung der Flüssigmetallmenge basierend auf einem Verhältnis der Amplitude und der Phase von dem gemessenen HF-Signal (S), und bevorzugt der angelegten HF-Wechselspannung, über ein Frequenzintervall erfolgt.

5. Messverfahren nach einem der vorangehenden Ansprüche, wobei die Frequenz der HF-Wechselspannung im Frequenzbereich VHF und UHF liegt.

6. Messverfahren nach einem der vorangehenden Ansprüche, wobei aus der gemessenen Induktivität des Flüssigmetall-Gleitlagers (6) oder einer mit der Induktivität zusammenhängenden Größe zunächst die Induktivität $L_{FM}$ des Flüssigmetallanteils ermittelt wird und dann mit vorbestimmten Induktivitätswerten $L_i$ von zumindest einem Strukurelement auf Basis der Formel

$$\frac{1}{L_{FM}} = \sum_{1}^{n} \frac{1}{L_i}$$

die Größe n ermittelt wird, wobei die Strukturelemente rein theoretische Elemente zur Beschreibung des Flüssigmetall-Gleitlagers (6) darstellen.

7. Messverfahren nach einem der vorangehenden Ansprüche, wobei vor der Ermittlung der Flüssigmetallmenge eine Kalibrierung an einem zum vermessenden Flüssigmetall-Gleitlager (6) baugleichen Flüssigmetall-Gleitlager (6) erfolgt, welches insbesondere vollständig gefüllt ist, bevorzugt mittels einer Durchleuchtung des baugleichen Flüssigmetall-Gleitlagers (6), besonders bevorzugt mittels eines Elektronenbeschleunigersystem-Scans.

8. Messverfahren nach einem der vorangehenden Ansprüche, wobei basierend auf der Ermittlung der Flüssigmetallmenge, insbesondere wenn die ermittelte Flüssigmetallmenge unter einem vorher festgelegten Grenzwert liegt, an dem Flüssigmetall-Gleitlager (6) eine weitere Messung der Flüssigmetallmenge mittels eines anderen Messverfahrens erfolgt, bevorzugt mittels einer Durchleuchtung des Flüssigmetall-Gleitlagers (6), besonders bevorzugt mittels eines Elektronenbeschleunigersystem-Scans.

9. Messvorrichtung (14) für ein Flüssigmetall-Gleitlager (6) mit zwei Lagerteilen (7, 8), zwischen denen Flüssigmetall (11) angeordnet ist, umfassend:

- eine Messeinheit (15) ausgelegt zur Messung eines HF-Signals (S) an elektrischen Kontakten (K1, K2) an unterschiedlichen Lagerteilen (7, 8), wobei das HF-Signal (S) von der Induktivität des Flüssigmetall-Gleitlagers (6) oder einer mit der Induktivität zusammenhängenden Größe abhängt,
- eine Ermittlungseinheit (16) ausgelegt zur Ermittlung der Flüssigmetallmenge in dem Flüssigmetall-Gleitlager (6) basierend auf dem gemessenen HF-Signal (S).

10. Messvorrichtung (14) nach Anspruch 9, umfassend eine HF-Einheit (12) ausgelegt zum Anlegen einer HF-Wechselspannung zwischen elektrischen Kontakten (K1, K2) an unterschiedliche Lagerteilen (7, 8) mit einem vorgegebenen Frequenzbereich.

11. Messvorrichtung (14) nach Anspruch 9 oder 10, umfassend eine Steuereinheit (17) ausgelegt zur Steuerung eines Messgeräts (18), welches ein anderes Messverfahren als die Messvorrichtung (14) durchführt, bevorzugt mittels einer Durchleuchtung des Flüssigmetall-Gleitlagers (6), besonders bevorzugt mittels eines Elektronenbeschleunigersystem-Scans.

12. Gerät, welches insbesondere zur Untersuchung mittels Röntgenstrahlung ausgelegt ist, umfassend eine Messvorrichtung (14) nach einem der Ansprüche 9 bis 11 und ein Flüssigmetall-Gleitlager (6), wobei das Flüssigmetall-Gleitlager (6) ein inneres Lagerteil (7) und ein äußeres Lagerteil (8) aufweist, zwischen welchen sich ein mit Flüssigmetall (11) gefüllter Lagerspalt (10) befindet.

13. Gerät nach Anspruch 12, umfassend

- eine Röntgenröhre mit einer Drehanode (1), wobei die Drehanode (1) mittels des Flüssigmetall-Gleitlagers (6) drehbar gelagert ist.

**Claims**

1. Measuring method for a liquid metal slide bearing (6) comprising the steps:

   - providing a liquid metal slide bearing (6) to be measured, which has two bearing parts (7, 8), between which the liquid metal (11) is arranged,
   - applying an RF alternating voltage between electrical contacts (K) to different bearing parts (7, 8) with a predetermined frequency range,
   - measuring an RF signal (S) on electrical contacts (K1, K2) on different bearing parts (7, 8), wherein the RF signal (S) depends on the inductance of the liquid metal slide bearing (6) or a variable associated with the inductance,
   - determining the quantity of liquid metal on the basis of the measured RF signal (S).

2. Measuring method according to claim 1, comprising the steps:

   - determining a resonance frequency ($RF$, $RF_L$, $RF_V$) from the RF signal (S),
   - determining the quantity of liquid metal on the basis of the resonance frequency ($RF$, $RF_L$, $RF_V$).

3. Measuring method according to one of the preceding claims, comprising the steps:

   - determining a minimum or a maximum of the bearing impedance from the measurement,
   - determining the quantity of liquid metal on the basis of the bearing impedance.

4. Measuring method according to one of the preceding claims, wherein the quantity of liquid metal is determined on the basis of a ratio of the amplitude and the phase of the measured RF signal (S), and the applied RF alternating voltage is preferably determined across a frequency interval.

5. Measuring method according to one of the preceding claims, wherein the frequency of the RF alternating voltage lies in the frequency range VHF and UHF.

6. Measuring method according to one of the preceding claims, wherein the inductance $L_{FM}$ of the liquid metal portion is determined from the measured inductance of the liquid metal slide bearing (6) or a variable associated with the inductance and the variable n is then determined with predetermined inductance values $L_i$ of at least one structural element on the basis of the formula

$$\frac{1}{L_{FM}} = \sum_{1}^{n} \frac{1}{L_i}$$

   wherein the structural elements represent purely theoretical elements for describing the liquid metal slide bearing (6).

7. Measuring method according to one of the preceding claims, wherein before determining the quantity of liquid metal, the liquid metal slide bearing (6) to be measured is calibrated to a liquid metal slide bearing (6) constructed in the same way, said liquid metal slide bearing being in particular completely filled, preferably by means of fluoroscopy of the liquid metal slide bearing (6) which is constructed in the same way, particularly preferably by means of an electron accelerator system scan.

8. Measuring method according to one of the preceding claims, wherein on the basis of the determination of the quantity of liquid metal, in particular if the determined quantity of liquid metal lies below a previously defined limit value, a further measurement of the quantity of liquid metal takes place on the liquid metal slide bearing (6) by means of another measuring method, preferably by means of fluoroscopy of the liquid metal slide bearing (6), particularly preferably by means of an electron accelerator system scan.

9. Measuring device (14) for a liquid metal slide bearing (6) with two bearing parts (7, 8), between which liquid metal (11) is arranged, comprising:

- a measuring unit (15) designed to measure an RF signal (S) on electrical contacts (K1, K2) on different bearing parts (7, 8), wherein the RF signal (S) depends on the inductance of the liquid metal slide bearing (6) or a variable associated with the inductance,
- a determination unit (16) designed for determining the quantity of liquid metal in the liquid metal slide bearing (6) on the basis of the measured RF signal (S).

10. Measuring device (14) according to claim 9, comprising an RF unit (12) designed to apply an RF alternating voltage between electrical contacts (K1, K2) to different bearing parts (7, 8) with a predetermined frequency range.

11. Measuring device (14) according to claim 9 or 10, comprising a control unit (17) designed for controlling a measuring apparatus (18), which carries out another measuring method than the measuring device (14), preferably by means of fluoroscopy of the liquid metal slide bearing (6), particularly preferably by means of an electron accelerator system scan.

12. Apparatus, which is designed in particular for examination by means of x-ray radiation, comprising a measuring device (14) according to one of claims 9 to 11 and a liquid metal slide bearing (6), wherein the liquid metal slide bearing (6) has an inner bearing part (7) and an outer bearing part (8), between which a bearing gap (10) filled with liquid metal (11) is located.

13. Apparatus according to claim 12, comprising

- an x-ray tube with a rotary anode (1), wherein the rotary anode (1) is rotatably mounted by means of the liquid metal slide bearing (6).

**Revendications**

1. Procédé de mesure d'un palier (6) lisse à métal liquide, comprenant les stades :

- on se procure un palier (6) lisse à métal liquide à mesurer, qui a deux parties (7, 8) de palier entre lesquels le métal (11) liquide est disposé,
- on applique une tension alternative HF entre des contacts (K) électriques sur des parties (7, 8) de palier différentes dans une plage de fréquences donnée à l'avance,
- on mesure un signal (S) HF sur des contacts (K1, K2) électriques sur des parties (7, 8) de palier différentes, dans lequel le signal (S) HF dépend de l'inductance du palier (6) lisse à métal liquide ou d'une grandeur en rapport avec l'inductance,
- on détermine la quantité de métal liquide sur la base du signal (S) HF mesuré.

2. Procédé de mesure suivant la revendication 1, comprenant les stades :

- on détermine une fréquence ($RF$, $RF_L$, $RF_V$) de résonnance à partir du signal (S) HF,
- on détermine la quantité de métal liquide sur la base de la fréquence ($RF$, $RF_L$, $RF_V$) de résonnance.

3. Procédé de mesure suivant l'une des revendications précédentes, comprenant les stades :

- on détermine un minimum ou un maximum de l'impédance du palier à partir de la mesure,
- on détermine la quantité de métal liquide sur la base de l'impédance du palier.

4. Procédé de mesure suivant l'une des revendications précédentes, dans lequel on effectue une détermination de la quantité de métal liquide sur la base d'un rapport de l'amplitude et de la phase du signal (S) HF mesuré et, de préférence, de la tension alternative HF appliquée, sur un intervalle de fréquence.

5. Procédé de mesure suivant l'une des revendications précédentes, dans lequel la fréquence de la tension alternative HF est dans la plage de fréquences VHF et UHF.

**6.** Procédé de mesure suivant l'une des revendications précédentes, dans lequel, à partir de l'inductance mesurée du palier (6) lisse à métal liquide ou d'une grandeur en rapport avec l'inductance, on détermine d'abord l'inductance $L_{FM}$ de la partie de métal liquide et on détermine ensuite la grandeur n par des valeurs $L_i$ d'inductance déterminées à l'avance d'au moins un élément de structure sur la base de la formule

$$\frac{1}{L_{FM}} = \sum_{1}^{n} \frac{1}{L_i}$$

les éléments de structures représentant des éléments purement théoriques de description du palier (6) lisse à métal liquide.

**7.** Procédé de mesure suivant l'une des revendications précédentes, dans lequel, avant la détermination de la quantité de métal liquide, on effectue un étalonnage sur un palier (6) lisse à métal liquide de même constitution que le palier (6) lisse à métal liquide à mesurer, qui est rempli, en particulier complètement, de préférence au moyen d'une transillumination du palier (6) lisse à métal liquide de même construction, en particulier, de préférence au moyen d'un scan de système d'accélérateur d'électrons.

**8.** Procédé de mesure suivant l'une des revendications précédentes, dans lequel, sur la base de la détermination de la quantité de métal liquide, en particulier, si la quantité de métal liquide déterminée est inférieure à une valeur limite fixée à l'avance, on effectue, sur le palier (6) lisse à métal liquide, une autre mesure de la quantité de métal liquide au moyen d'un autre procédé de mesure, de préférence au moyen d'une transillumination du palier (6) lisse à métal liquide, en particulier, de préférence au moyen d'un scan de système d'accélérateur d'électrons.

**9.** Dispositif (14) de mesure d'un palier (6) lisse à métal liquide ayant deux parties (7, 8) de palier entre lesquelles est disposé du métal (11) liquide, comprenant :

- une unité (15) de mesure conçue pour la mesure d'un signal (S) HF sur des contacts (K1, K2) électriques sur des parties (7, 8) de palier différentes, dans lequel le signal (S) HF dépend de l'inductance du palier (6) lisse à métal liquide ou d'une grandeur en rapport avec l'inductance,
- une unité (16) de détermination conçue pour la détermination de la quantité de métal liquide dans le palier (6) lisse à métal liquide, sur la base du signal (S) HF mesuré.

**10.** Dispositif (14) de mesure suivant la revendication 9, comprenant une unité (12) HF conçue pour l'application d'une tension alternative HF entre des contacts (K1, K2) électriques sur des parties (7, 8) de palier différentes dans une plage de fréquences donnée à l'avance.

**11.** Dispositif (14) de mesure suivant la revendication 9 ou 10, comprenant une unité (17) de commande conçue pour la commande d'un appareil (18) de mesure, qui effectue un autre procédé de mesure que le dispositif (14) de mesure, de préférence au moyen d'une transillumination du palier (6) lisse à métal liquide, en particulier, de préférence au moyen d'un scan de système d'accélérateur d'électrons.

**12.** Appareil, qui est conçu, en particulier pour l'examen au moyen du rayonnement X, comprenant un dispositif (14) de mesure suivant l'une des revendications 9 à 11 et un palier (6) lisse à métal liquide, dans lequel le palier (6) lisse à métal liquide a une partie (7) de palier intérieure et une partie (8) de palier extérieure entre lesquelles se trouvent une fente (10) de palier remplie de métal (11) liquide.

**13.** Appareil suivant la revendication 12, comprenant

- un tube à rayons X ayant une anode (1) tournante, dans lequel l'anode (1) tournante est montée tournante au moyen d'un palier (6) lisse à métal liquide.

FIG 1

FIG 2

EP 3 792 621 B1

**FIG 3**

**FIG 4**

FIG 5